# FASCICULE DE BREVET EUROPEEN

(11) **EP 1 390 751 B1**
(45) Date de publication et mention de la délivrance du brevet: **30.11.2011**
(21) Numéro de dépôt: 02740818.6
(22) Date de dépôt: 27.05.2002
(51) Int. Cl.: G01N 33/543, G01N 33/68

(54) **DISPOSITIF DE PRESENTATION DE POLYPEPTIDES, UTILISABLE COMME "PUCE" POUR LA DETECTION MINIATURISEE DE MOLECULES.**
VORRICHTUNG ZUR POLYPEPTIDPRÄSENTATION, VERWENDBAR ALS SENSORCHIP ZUR MINIATURISIERTEN DETEKTION VON MOLEKÜLEN
DEVICE FOR PRESENTING POLYPEPTIDES, ABLE TO BE USED AS A CHIP FOR MINIATURISED DETECTION OF MOLECULES

(30) Priorité: 28.05.2001 FR 0106931
(43) Date de publication de la demande: 25.02.2004
(73) Titulaire: CENTRE NATIONAL DE LA RECHERCHE SCIENTIFIQUE (CNRS), 75016 Paris (FR); UNIVERSITE DE LILLE II, 59800 Lille (FR)
(72) Inventeur: MELNYK, Oleg, F-59000 Lille (FR); GRAS-MASSE, Hélène, F-59000 Lille (FR); OLIVIER, Christophe, F-59000 Lille (FR); DURAND, Jean-Olivier, F-59000 Lille (FR); AURIAULT, Claude, F-59000 Lille (FR); DUBURCQ, Xavier, F-59000 Lille (FR); BOUZIDI, Ahmed, F-59000 Lille (FR); GARCIA, Jean-Michel, F-59800 Lille (FR); EL-MAHDI, Ouafâa, F-59800 Lille (FR)
(74) Mandataire: Gutmann, Ernest
(86) Numéro de dépôt international: PCT/FR2002/001771
(87) Numéro de publication internationale: WO 2002/097442

(56) Documents cités:
- WO-A-00/36419
- WO-A-01/09385
- WO-A-93/12076
- US-A- 5 849 480
- US-A- 5 877 278

## Description

L'invention concerne un dispositif de présentation de peptides ou de protéines, qui est utilisable comme « puce à polypeptides » pour la détection miniaturisée de molécules structurellement ou fonctionnellement complémentaires desdits polypeptides. Ce dispositif consiste en un support plat sur lequel les polypeptides sont liés de manière covalente, cette liaison entre les polypeptides et le support résultant de la formation d'un lien semicarbazone, qui résulte en particulier de la réaction entre
- des polypeptides portant une fonction aldéhyde ou cétone
- et un support fonctionnalisé par des groupements semicarbazides, et en ce que les fonctions aldéhyde ou cétone portées par les polypeptides sont des α-oxoaldéhide ou α-oxocétone, et sont situées du côté C-terminal ou du côté N-terminal, ou sur une chaîne latérale.

On utilisera ci-après le terme général de « polypeptide » pour désigner des peptides (comprenant au moins 2 acides aminés, 1 qui peuvent être de la série L ou D, des alpha-aminoacides, des beta-aminoacides, des alpha-hydrazinoacides, des alpha-aminoacides protéinogéniques ou non), des peptidomimétiques (mimes de structure secondaire, mimes de coude beta par exemple), et des protéines ou fragments de protéines.

Le terme de « puce à polypeptides » correspond aux termes anglais « peptide arrays, peptide microarrays ou peptide chips », courants dans la littérature.

L'invention concerne également le procédé de préparation des supports et de fixation des polypeptides sur ces supports ainsi que leur utilisation comme puces à polypeptides.

De telles puces sont particulièrement favorables à la détection, dans divers milieux biologiques liquides, d'anticorps ou de parties spécifiques de ceux-ci, d'antigènes (notamment viraux, bactériens ou parasitaires), de récepteurs, de séquences responsables de la liaison à une molécule (enzyme, récepteur, anticorps), à l'étude de la spécificité d'enzymes, à la mise au point de récepteurs artificiels...

Les biopuces ont d'abord été mises au point et développées pour la détection d'acides nucléiques. Des produits similaires à base de polypeptides font l'objet de nombreuses recherches mais ne sont pas encore optimisés. C'est donc surtout dans les publications et les brevets concernant les puces à ADN qu'on trouve l'art antérieur pertinent.

Hormis la technologie de photolitographie d'Affymetrix et de jet d'encre de ProtoGene, qui mettent en jeu la synthèse d'oligonucléotides in situ, la fabrication de « microarrays » utilise le plus souvent des « spotters » à aiguilles et des lames de verre comme supports. Les aiguilles prélèvent les sondes préparées dans des puits de plaques de microtitration et viennent frapper la surface du matériau qui sert de support.
Il est maintenant communément admis que les microarrays. à ADN élaborés par dépôt de sondes sur un support souffrent de nombreuses limitations. Certaines sont liées à l'appareillage lui-même (reproductibilité du volume déposé), mais la plupart sont la conséquence de la chimie organique/inorganique et d'interface mise en jeu : bruit de fond trop important, sensibilité, rendement d'immobilisation, dégradation des sondes due aux différents traitements nécessaires à l'immobilisation, stabilité du lien sonde-support lors des différents lavages et lors de l'étape de recyclage du microarray, qualité du support (uniformisé du traitement, stabilité dans le temps), adsorption non spécifique sur le support, faux positifs.

En plus de ces problèmes connus, liés à la fixation des nucléotides, la conception de puces à polypeptides comporte des problèmes spécifiques dus à l'existence, sur les polypeptides, de diverses fonctions réactives situées sur des chaîne latérales des acides aminés qui peuvent interférer avec les méthodes d'immobilisation, ce qui entraîne la fixation par des liens multiples ou la formation de liaisons latérales entre polypeptides, ce qui risque de diminuer la réactivité des polypeptides fixés vis-à-vis des liquides biologiques qui seront testés sur ces puces.

Les méthodes mises au point pour fabriquer des puces à nucléotides ne sont donc pas directement transposables à la fabrication des puces à polypeptides, celles-ci nécessitant la mise en oeuvre de méthodes de couplage chimiosélectives.

Diverses méthodes de couplage plus ou moins prometteuses ont déjà été décrites.
- Ainsi, la chimie des thiols a été utilisée mais elle présente des limitations importantes : oxydation des thiols lors de leur préparation, de leur conservation et de leur dépôt sur les supports (problème d'autant plus important que lors du dépôt, la surface de contact du film avec l'air est importante). Par exemple, cette chimie a été utilisée pour ancrer des peptides de type RGD sur de la silice (Porté-Durrieu, MC et al. J. Colloid Interface Science 1990, 134, 368-375), ou des anticorps sur du quartz (Weiping, Q. et al. Supramolecular Science 1998, 5, 701-703, formation de disulfures). On peut également citer la formation de thioéthers, et la formation de liaison Hg-S (Rao, S.V. et al. Mikrochim. Acta 1998, 128, 127-143).
- De nombreux laboratoires ont immobilisé des protéines via la formation d'un lien hydrazone entre un aldéhyde et un hydrazide. Cependant, ces méthodologies présentent aussi de nombreuses limitations. Le lien hydrazone n'est pas très stable et s'hydrolyse rapidement (King, TP et al. Biochemistry 1986, 25, 5774-5579). Pour cette raison, de nombreux auteurs réduisent ce lien avec un borohydrure pour stabiliser la liaison (voir par ex King, TP et al. Biochemistry 1986, 25, 5774-5579, Ruhn, P.F. et al. J. Chromatography A 1994, 669, 9-19). D'autre part, la réaction d'un hydrazide avec un aldéhyde est relativement lente.
- Falipou, S. et al. (Bioconjugate Chem 1999, 10, 346-53) décrivent la silanisation de billes de SiO2 ou de lames de verre avec du 3-cyanopropyldimethylchlorosilane. Les surfaces ainsi traitées permettent d'immobiliser des anticorps (via les hydroxyles des parties glycosylées) de façon non covalente.
- Dans le brevet US4874813, il est décrit que le couplage d'IgG (après oxydation periodique pour générer une fonction aldéhyde) à un support hydrazide s'effectue avec un rendement de 53% seulement. Cette chimie n'est pas bien adaptée à la technologie biopuces, où les réactions doivent être très rapides pour compenser la faible concentration de substance à fixer et sa mobilité au cours du séchage du dépôt. Dans le même brevet, il est décrit que l'incorporation au niveau du support d'une amine tertiaire (pKa<8) permet d'accélérer la liaison au support. Il s'agit typiquement d'une accélération due aux interactions non spécifiques induites par l'ammonium. Ce phénomène est bien discuté par Robberson et al. (Biochemistry 1972, 11, 533-536) dans le cadre de l'immobilisation d'ARN sur un gel d'agarose hydrazide, et est probablement dû à la modification des propriétés de diffusion des biomolécules suite à la réduction de dimensionnalité (Adam, G. et al. 1968, in Structural Chemistry and Molecular Biology, Rich, A. Davidson, N. Ed., San Francisco, WH Freeman). Cependant une telle adsorption non spécifique est justement ce qu'il faut éviter au maximum dans le cadre des biopuces.
- L'immobilisation d'IgG oxydée au periodate sur une silice hydrazide est décrite par Ruhn, AF et al. (J. Chromatography A 1994, 669, 9-19). La réaction prend 1 jour à 4°C ; ce support se décompose à plus haute température et donne lieu à une adsorption non spécifique importante qui s'élève à 20% de la valeur obtenue par liaison covalente. Ce type de chimie n'est pas favorable pour la réalisation de biopuces.
- Dans le brevet US 4801726, il est décrit un support (silice) hydrazide pour l'immobilisation de toxines de nature non peptidique par un lien non hydrazone. Le support (uniquement des gels de silice, pas de surfaces planes) est préparé par réaction avec un silane époxyde puis ouverture de l'époxyde avec un dihydrazide. Or, la réaction de dinucléophiles sur un support est connue pour donner de nombreuses réactions de pontage. Ce point est bien documenté dans l'article de Ruhn, PF et al. J. Chromatrography A 1994, 669, 9-19. Ainsi, la densité de fonction de surface est fortement diminuée. Les réactions de pontage sont également la source de problèmes de reproductibilité.
- Plusieurs travaux font appel à la synthèse in situ des polypeptides sur le support :
   - Ashfield, C. et al. " Synthesis of peptides in picoliter virtual flasks " (16th American Peptide Symposium Minneapolis, Minnesota, june 26-july 1, 1999 Poster 823) décrivent la synthèse in situ de peptides sur semiconducteur. Des électrodes sont couvertes d'un polymère poreux, au sein duquel s'effectue la synthèse peptidique. Un linker comportant une fonction -NH-Fmoc est lié au polymère. Le groupement Fmoc est enlevé en générant *in situ* une base grâce à l'application d'un potentiel redox. La fonction amine ainsi libérée est mise en réaction avec des acides aminés activés de façon traditionnelle.
   - Pellois, J. P. et al. (J. Comb. Chem. 2000, 2, 355-360) décrivent la synthèse in situ sur lame de verre.
La chimie de base est de type Boc/benzyl. La lame de verre est silanisée amine, puis le premier acide aminé est couplé de manière traditionnelle. La déprotection du groupement tBoc s'effectue par irradiation (400 nm) en présence de triarylsulfonium hexafluoroantimonate ou de diaryl iodium hexafluoroantimonate. Le couplage de l'acide aminé suivant est fait de manière traditionnelle. • Dans le Brevet WO 0053625, 1 un mode de synthèse in situ de biopuce est décrit.
Un polymère poreux est déposé sur une série d'électrodes. Dans un premier temps, un Fmoc-acide aminé-OH est couplé au polymère poreux. Le groupement Fmoc est enlevé grâce à l'application d'un potentiel sur une électrode donnée en présence d'azobenzène. La biopuce est ensuite immergée dans une solution contenant l'acide aminé suivant activé.

La synthèse in situ souffre de nombreuses limitations :
- elle nécessite l'utilisation de groupements protecteurs photolabiles ou de réactifs activés par l'application d'un potentiel électrique ;
- cette technologie est peu efficace actuellement (limitation de la taille des peptides, coût élevé, peu flexible - cf commentaires de Pellois, J.P. J. Comb. Chem. 2000, 2, 355-360),
- la caractérisation des peptides liés au support est difficile.
Dans un tout autre domaine, l'utilisation de billes de résines de polystyrène fonctionnalisées par des fonctions semicarbazides a été décrite pour réaliser la synthèse, pas à pas, de peptides aldéhydes :
- Siev, DV et al. (Org. Lett 2000, 2, 19-22) décrivent une résine polystyrène hydrazino-carbonyl-aminomethylé (billes) pour la synthèse de peptides et peptidomimétiques aldéhyde.
- Patterson, JA et al. (Tetrahedron Lett. 1999, 40, 6121-6124) décrivent, à partir d'une résine de type polystyrène aminé, la formation de l'isocyanate avec du triphosgène et la réaction de l'isocyanate avec Fmoc-NH-NH2 (préparée selon Fhang, Z.E. et al. Anal. Biochem. 1991, 195, 160-170). Le support sert à l'immobilisation d' aldéhydes aminés, puis à la synthèse en phase solide de peptides aldéhydes protégés sous forme de semicarbazides. La semicarbazone est échangée en solution par de l'acide pyruvique pour libérer le peptide aldéhyde.
- Murphy, AM et al. (J. Am. Chem. Soc. 1992, 114, 3156-3157) décrivent la réaction d'un aldéhyde aminé en phase homogène avec un semicarbazide fonctionnalisé par une fonction acide carboxylique. Après la formation de la semicarbazone, le synthon est ancré à un support solide (billes) et sert à la synthèse de peptides aldéhydes en phase solide.

Un progrès significatif dans l'élaboration de biopuce à peptides a été présenté par Falsey et al. (16th American Peptide Symposium - Minneapolis 1999 - Poster n° 822).
Ces auteurs ont lié des peptides fonctionnalisés par une hydroxylamine H2N-O- ou un beta-aminothiol (cystéine N-terminale) à des lames de verre fonctionnalisées par une fonction alpha-oxoaldéhyde. La lame de verre a été silanisée amine, puis les fonctions amines ont été dérivatisées par une Fmoc-Ser-OH. Après déprotection du groupement Fmoc à la pipéridine, les lames ont été traitées par du periodate de sodium (oxydation du beta-aminoalcool en alpha-oxoaldéhyde) L'inconvénient majeur de cette méthodologie est l'adsorption non spécifique de polypeptides. à la surface, menant à un bruit de fond important.

Les Demanderesses ont cherché à résoudre les principales limitations des dispositifs développés précédemment, et en particulier :
- les problèmes de bruit de fond dus à l'adsorption non spécifique des échantillons à tester ;
- la fonctionnalisation trop faible ou irrégulière de la surface du support ;
- l'ancrage peu efficace des sondes (polypeptides).

La demande de brevet WO 96/40263 divulgue la liaison d'un composant à un support solide via une liaison qui est décrite comme étant de type semicarbazone (*cf*., par exemple, de page 6 ligne 20 à page 7 ligne 11 de WO 96/40263).

Ainsi, les dispositifs selon l'invention comprennent des supports plats, en matériau solide, organique ou inorganique qui peuvent être par exemple en verre, en silicium en polymère naturel ou de synthèse..., présentant une surface plane fonctionnalisée par un groupement tel qu'un groupement semicarbazide pour immobiliser de façon contrôlée des polypeptides choisis en tirant parti de la formation d'une liaison semicarbazone. Celle-ci est obtenue par l'utilisation de polypeptides préalablement fonctionnalisés par un groupement alpha-oxoaiddhyde ou alpha-oxocétone.

Dans le cas de polypeptides de synthèse, le groupement alpha-oxo aldéhyde ou alpha-oxo cétone est introduit en cours de synthèse soit à l'extrémité N-terminale, soit à l'extrémité C-terminale, ou sur une chaîne latérale (de Lys ou Cys).
On décrit également pour le cas de polypeptides naturels glycosylés qu'on peut génèrer des groupements alpha-oxo aldéhyde par oxydation des résidus polysaccharidiques.
Dans le cas de polypeptides (fragments de protéines) naturels (glycosylée ou non) on peut générer une fonction alpha-oxo cétone ou alpha-oxo aldéhyde par transamioetion du résidu N-terminal ou une fonction alpha-oxo aldéhyde ou alpha-oxo cétone grâce à l'utilisation d'un agent bifonctionnel.
La fonction alpha-oxo aldéhyde ou alpha-oxo cétone peut être située sur un bras espaceur.

Le dépôt des polypeptides fonctionnalisés sur le support semicarbazide n'accompagne de la liaison spontanée du peptide au support, dans des conditions de pH, de température et d'humidité choisies.

Les lames semicarbazides peuvent être imprimées par exemple avec un « spotter » à aiguilles. Après le dépôt des polypeptides sur la lame, celle-ci peut être trempée, lorsque cela est nécessaire, dans une solution contenant un polyéthylèneglycol dérivatisé par une fonction α-oxoaldéhyde ou alpha-oxo cétone. Ainsi, tous les sites réactifs présents entre les spots sont liés de manière covalente à un PEG, via le même lien semicarbazone utilisé : pour immobiliser les polypeptides, ce qui réduit encore l'adsorption non spécifique des échantillons-tests.
Ainsi, la stratégie d'immobilisation
- est simple au niveau expérimental et d'une grande reproductivité ;
- s'applique aux peptides polyfonctionnels, y compris les polypeptides comprenant des cystéines ;
- met en jeu des polypeptides modifiés par une fonction stable et facile à introduire ;
- met en jeu des surfaces fonctionnalisées par une fonction stable, non hydrolysable ;
- fait intervenir des fonctions très réactives, compensant la faible concentration des polypeptides au niveau du dépôt ;
- permet d'obtenir une grande densité de fixation de surface ce qui assurera un rapport signal sur bruit de fond très élevé ;
- respecte la structure du polypeptide (liaison spécifique au support par un seul point d'ancrage) ;
- permet un contrôle qualité de toutes les étapes.

La chimie de ligation hydrazone a été fortement développée dans le domaine de l'ingénierie des protéines pour la synthèse convergente de macromolécules grâce à la liaison contrôlée de fragments totalement déprotégés et purifiés. Les deux groupements fonctionnels, présents sur chacun des fragments, 1 se reconnaissent mutuellement avec une grande sélectivité dans des conditions très douces (voir par exemple J.P. Tam et al. Biomed. Peptide Protein & Nucleic Acids 1995, 1, 123-132). Les liaisons semicarbazones sont beaucoup plus stables que les hydrazones et ont été choisies pour cette raison pour la fixation des polypeptides selon la présente invention.

De plus il est apparu, de manière surprenante, que la liaison des groupements semicarbazides sur le support se réalisait de manière dense, homogène et reproductible. Cette densité de fonctionnalisation est apparue comme une des clefs de la réussite des biopuces selon l'intention en effet elle augmente la sensibilité du test ce qui est essentiel pour travailler avec des microquantités et permet ainsi de mesurer des réponses (sonde-cible, antigène-anticorps...) sans avoir recours à des artifices d'amplification. Elle assure également un bruit de fond extrêmement faible et donc un rapport signal sur bruit de fond très élevé ce qui augmente la sensibilité du test et sera particulièrement favorable dans le cas de teste mettant en oeuvre des liquides biologiques très riches en protéines diverses.

La qualité de la fonctionnalisation du support selon l'invention (densité et homogénéité) peut être contrôlée par sa capacité de fixer une sonde peptidique de synthèse, fluorescente, dérivatisée par une fonction α-oxoaldéhyde.

L'invention concerne également le procédé de préparation des dispositifs de présentation des polypeptides qui comprend les étapes suivantes :
1- introduction d'une fonction alpha-oxo aldéhyde ou alpha-oxo cétone, par synthèse ou par modification d'une fonction naturelle, à l'une des extrémités N ou C ou sur une chaîne latérale d'un polypeptide de synthèse ou naturel ;
2- fonctionnalisation d'un support solide par des groupements semicarbazides ;
3- dépôt sous forme de spots d'échantillons de polypeptides obtenus par l'étape 1 sur le support fonctionnalisé de l'étape 2, dans des conditions de pH et d'humidité assurant la réaction entre la fonction alpha-oxo aldéhyde ou alpha-oxo cétone et la fonction semicarbazide pour créer le lien semicarbazone.

L'étape 1 peut être effectuée en cours de synthèse d'un polypeptide à l'aide d'un synthétiseur automatique. Elle peut comprendre l'introduction, d'un bras espaceur entre le dernier acide aminé de la séquence du polypeptide et la fonction alpha-oxo aldéhyde ou alpha-oxo cétone.

L'étape 1 peut être effectuée par oxydation d'un polysaccharide d'une glycoprotéine naturelle (pas partie de l'invention) ou d'un fragment de celle-ci ou par transamination d'un acide aminé N-terminal d'une protéine naturelle non glycosylée ou d'un fragment de celle-ci, ou par l'action d'un agent bifonctionnel.

L'étape 2 comprend
- une réaction de silanisation du support, introduisant une fonction amine ;
- la transformation de la fonction amine en fonction isocyanate ;
- la réaction de la fonction isocyanate avec un dérivé d'hydrazine pour former le groupement semicarbazide.

Alternativement, l'étape 2 peut être effectuée en une seule réaction d'un silane portant un groupement semicarbazide qui est, de préférence protégé par le Fmoc.

L'étape 3 comprend de préférence
- la préparation de solutions des polypeptides de l'étape 1 à 10⁻³ ou 10⁻⁴M dans un tampon acétate 0,1M à pH 5,5,
- leur distribution dans un récipient approprié à leur prélèvement, de type puits de plaque de microtitration,
- leur prélèvement à l'aide d'un « spotteur »,
- et leur dépôt sur le support semicarbazide ;
- l'incubation des lames pendant une nuit à 37°C sous atmosphère humide
- et leur lavage et la saturation des sites réactifs non spécifiques.

L'invention concerne également l'utilisation des dispositifs de présentation des polypeptides comme « puces à polypeptides » en tant qu'outil de diagnostic.
Cette utilisation comprend la détection des réponses de type antigène-anticorps par l'utilisation de réactifs marqués, fluorescents, radioactifs ou marqués chimiquement, comme dans les tests de diagnostic non miniaturisés.

Les dispositifs selon l'invention peuvent également être utilisés comme puces à polypeptides pour le criblage de molécules et pour l'analyse des relations entre molécules, de type ligand-récepteur.

Les exemples qui suivent illustrent l'invention sans toutefois en limiter la portée. Les figures suivantes y sont annexées :
Figure 1 : test comparatif de sérums sur le peptide HCVpc21-2 en ELISA (mesure de la DO) et en lames semicarbazides (mesure de la fluorescence/20000).
Figure 2 : étude de la corrélation entre les mesures de la DO et de la fluorescence, présentées dans la figure 1.
Figure 3 : test comparatif de sérums sur 3 peptides HCVpc21 « petit linker », « grand linker » et N-terminal (HCVpc21 3. 2 et 1) ; mesure de la fluorescence.
Figure 4 : comparaison des réponses de 30 sérums positifs avec les peptides HCVpc21, NS4 et un mélange pc21/NS4 (1 pour 10) ; mesure de la fluorescence.

### Exemple 1 - Fonctionnalisation de lames de verre

La liaison covalente de groupements semicarbazides sur des lames de verre a été réalisée selon 2 stratégies.

### Première variante

### Etape A : lavage, décapage et silanisation

Des lames porte-objets commerciales (Esco) prénettoyées, à bords rodés et à marge dépolie sont plongées dans une solution « piranha » (eau oxygénée/acide sulfurique, 50/50) pendant une nuit. Des rinçages préalables de trois minutes sont effectués par de l'eau desionisée (3 fois) puis par du méthanol (1 fois), avant de plonger les lames dans un bain à 3% d'aminopropyl-triméthoxysilane dans du méthanol à 95% pendant 30 minutes aux ultrasons. Les lames sont rincées successivement par des bains de 3 minutes dans du méthanol (1 fois), par de l'eau, desionisée (2 fois) et .enfin par du méthanol (1 fois). Les lames sont ensuite égouttées pendant quelques minutes, séchées 15 minutes dans une étuve à 110°C puis stockées dans un dessiccateur sous vide.

### Etape B : formation d'un isocyanate

Les lames précédemment silanisées sont plongées pendant 2 heures dans une solution de 1,2-dichloroéthane contenant du triphosgène (100 mmol/l) et de la DIEA (800 mmol/l).

### Etape C : fonctionnalisation par un semicarbazide.

Ces lames sont ensuite rapidement égouttées avant d'être directement plongées dans la solution contenant le Fmoc-NH-NH₂ (préparé selon Zhang et al. Anal. Biochem. 1991, 195, 160-170) à 22 mmol/l dans le DMF et traitées pendant 2 heures aux ultrasons.
Les lames sont ensuite rincées successivement par deux bains de 3 minutes dans du DMF.

### Etape D : déprotection

Les lames précédemment obtenues sont plongées dans une solution de DMF contenant de la pipéridine (0,2% en volume) et du diazabicyclo-undecène (2% en volume) pendant 30 minutes. Les lames sont alors rincées successivement par des bains de 3 minutes dans du DMF (1 fois), par de l'eau desionisée (2 fois) et enfin par du méthanol (1 fois) avant d'être séchées et stockées dans un dessiccateur sous vide.

### Deuxième variante

Les étapes de silanisation et de fonctionnalisation semicarbazide sont couplées en une seule réaction, par préparation préalable du réactif.

Etape A :préparation du réactif Fmoc-NH-NH-CO-NH-(CH₂)₃-Si (OEt)₃).

La préparation du réactif est effectuée selon le schéma suivant :

### 1°) Préparation du réactif

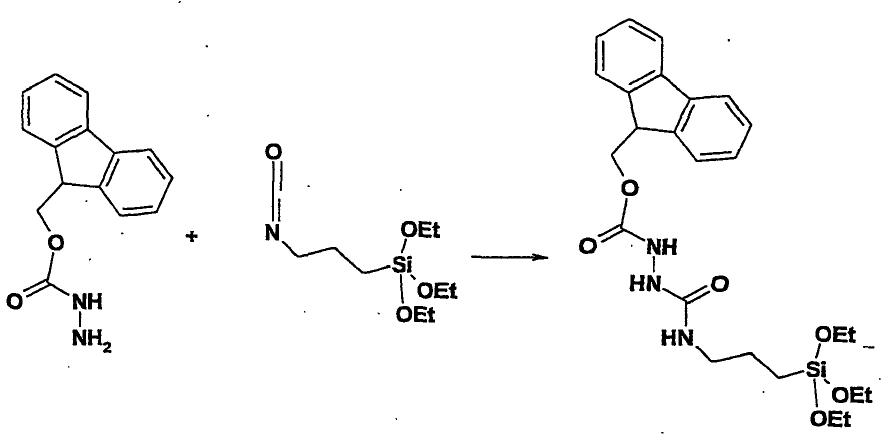

515 mg de Fmoc-NHNH₂ (2.03 mmoles) sont mis en suspension dans 15 ml d'éthanol absolu. Le mélange est porté au reflux (75-80°C). 570 ml d'isocyanopropyltriethoxysilane (2,28 mmoles, 1,2 eq) sont alors ajoutés en une fois. Après disparition de la suspension (15-20 minutes), l'ethanol, est évaporé. Le solide blanc est dissous dans un minimum de dichlorométhane sec, puis précipité grâce à du pentane sec. Après filtration sous argon, on récupère 841 mg (83%) d'un solide pur.

### Etape B :préparation des lames

Des lames porte-objets (Esco) prénettoyées, à bords rodés et à marge dépolie sont plongées pendant une nuit sous agitation dans une solution fraîchement préparée de piranha (H₂SO₄/H₂O₂). Les lames sont ensuite rincées sous agitation dans les bains successifs suivants : eau desionisée (3 fois 3 minutes), éthanol absolu (1 fois 3 minutes) puis séchées à la pompe à palettes.
Elles sont ensuite plongées pendant 2 heures dans une solution à 1mg/ml de réactif de silanisation (Fmoc-NH-NH-CO-NH-(CH₂)₃-Si(OEt)₃) dans un mélange de THF à 10% dans du toluène à 47°C et sous ultrasons. Les lames sont ensuite rincées sous agitation dans du toluène (2 fois 3 minutes) avant d'être égouttées, puis séchées 15 minutes dans une étuve à 120°C et stockées dans un dessiccateur sous vide.

### Etape C : déprotection

Les lames précédemment obtenues sont plongées dans une solution de DMF pendant 3 minutes avant d'être mises sous agitation dans un bain contenant de la pipéridine (0,2% en volume) et du diazabicyclo-undecène (2% en volume) dans du DMF pendant 30 minutes sous agitation. Les lames sont alors rincées successivement par des bains de 3 minutes dans du DMF (1 fois 3 minutes), dans de l'eau désionisée (2 fois 3 minutes) et enfin dans du méthanol (1 fois 3 minutes), avant d'être séchées et stockées dans un dessiccateur sous vide.

### Exemple 2 - Contrôle de qualité des lames semicarbazide

Il est important de vérifier que la fonctionnalisation des lames est homogène et reproductible pour garantir une fixation homogène des polypeptides.

La qualité des lames est donc contrôlée en utilisant la même réaction que celle qui sera utilisée pour fixer les polypeptides. On utilise un petit peptide de synthèse fonctionnalisé par un groupe α-oxoaldéhyde et marqué à la rhodamine (marqueur fluorescent), et, comme contrôle négatif, le même peptide mais dans lequel la fonction α-oxoaldéhyde est remplacée par un groupe amide.

Le peptide rhodaminé fonctionnalisé par un groupe α-oxoaldéhyde de séquence (5)-6-carboxytetraméthylrhodamine-Lys-Arg-NH-(CH₂)₃-NH-CO-CHO a été synthétisé à partir du linker IPT (2,3-O-isopropylidène-D-tartrate), décrit par J.S. Fruchart et al. H. Gras-Masse, O. Melnyk (A new linker for the synthesis of C-terminal peptide α-oxoaldéhydes ; Tet. Lett. ; 40 ; 6225-6228-1999), et dans la demande de brevet PCT/FR00/01035.
On synthétise comme peptide de contrôle : (5)-6-carboxytétraméthyl-rhodamine-Lys-Arg-NH₂.

### Révélation :

Les lames fonctionnalisées par un groupe semicarbazide sont trempées pendant 1 heure à 37° C dans un bain du peptide rhodaminé (fonctionnalisé du coté C-terminal par un α-oxoaldéhyde ou témoin non fonctionnalisé) à une concentration de 0,1 mM dans un tampon acétate 100 mM est à pH 5,5. Les lames sont ensuite rincées par un passage dans un bain d'eau desionisée. Elle sont ensuite transvasées dans une solution de K₂HPO₄ à 5% dans l'eau pendant 2 heures et traitées aux ultrasons. Les lames sont ensuite rincées par des bains sous agitation d'eau desionisée (2 fois 3 minutes) puis sont trempées pendant 30 minutes aux ultrasons dans une solution de Tris-acétate (100mM) à pH 5,5 en présence de Tween 20 (0,1 %). Les lames sont ensuite rincées successivement par bains dans de l'eau desionisée (2 fois 3 minutes) et enfin dans du méthanol (1 fois 3 minutes). Les lames sont ensuite séchées dans un dessiccateur sous vide.

Les lames sont ensuite passées au scanner MWG (L30 PMT45). On quantifie la fluorescence de l'ensemble de la lame en utilisant une grille de 32 lignes et 10 colonnes de spots sur le logiciel ScanAlyse. Les valeurs de fluorescence sont présentées dans le tableau suivant :

| Type de lame | Lames fonctionnalisées | | | | Lames non fonctionnalisées | |
|---|---|---|---|---|---|---|
| | Première variante | | Deuxième variante | | | |
| Type de sonde | α-oxo-aldéhyde | amide | α -oxo-aldéhyde | amide | α -oxo-aldéhyde | amide |
| Fluorescence moyenne | 36260 | 3450 | 28882 | 1732 | 7789 | 5270 |
| Ecart type | 3939 | 486 | 3427 | 91 | 671 | 934 |

### Exemple 3 - Synthèse de peptides tests

Les peptides suivants ont été synthétisés (sur un synthétiseur automatique de type Pioneer PerSeptive Biosystem).

### 1 - Peptides du virus de l'hépatite C

- Peptide **HCVpc21-1** fonctionnalisé par un groupe α-oxoaldéhyde du côté N-terminal : 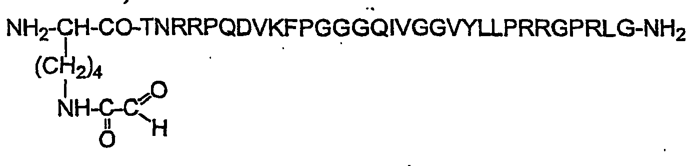 Ce peptide est synthétisé en phase solide depuis l'extrémité C-terminale vers l'extrémité N-terminale selon une stratégie Fmoc, sur une résine Fmoc-PAL-PEG-PS (Perseptive Biosystems).
- Peptide **HCVpc21-2** fonctionnalisé par un groupe 4,7,10-trioxa-1,13-diamino-tridecanyl-α-oxoaldéhyde du côté C-terminal, " grand linker " :

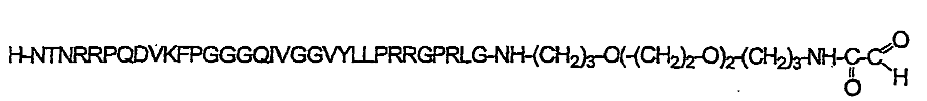

Ce peptide est synthétisé en phase solide depuis l'extrémité C-terminale vers l'extrémité N-terminale selon une stratégie Fmoc sur une résine « méthyl-2,3-O-isopropylidène-D-tartryl-Val-PEGA, préparée comme décrit dans « Peptides for the new millenium », proceeding of the 16th american peptide symposium, (kluwer academic publishers, Dordrecht, 2000, p104-106).
0,1 mmole de résine sont conditionnés dans un réacteur de phase solide par des lavages au DCM (2 fois 2 minutes) puis au DMF (2 fois 2 minutes). On additionne alors sur la résine presque sèche un mélange de 1,688 ml de 4,7,10 Trioxa-1,13-tridecane-diamine (7,7 mmoles) et de DFM (812 µl). Après 45 minutes d'agitation, la résine est lavée par deux lavages successifs au DMF. Le premier aminoacide de la séquence est couplé dans le réacteur et on additionne à la résine presque sèche 10 eq des réactifs suivants : Fmoc-Gly-OH, HBTU, HOBt et 30 eq de DIEA dans du DMF (2,5ml). La solution est agitée pendant 45 minutes avant d'être lavée au DMF (4x2min) puis au DCM (4x2min). Les aminoacides suivants sont alors greffés en transférant la résine obtenue sur un synthétiseur automatique de peptide de type Pioneer PerSeptive Biosystem.
- Peptide **HCVpc21-3** fonctionnalisé par un groupe 1-3-diaminopropyl-α-oxoaldéhyde du côté C-terminal " petit linker " :

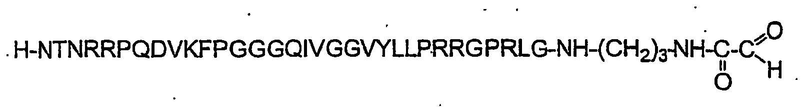

Ce peptide est synthétisé en phase solide depuis l'extrémité C-terminale vers l'extrémité N-terminale selon une stratégie Fmoc sur une résine méthyl-2,3-O-isopropylidène-D-tartryl-Val-PEGA.
0,1 mmole de résine sont conditionnés dans un réacteur de phase solide par des lavages au DCM (2 fois 2 minutes) puis au DMF (2 fois 2 minutes). On additionne alors sur la résine presque sèche un mélange de 649 µl de 1,3 diaminopropane (7,7 mmoles) et de DMF (351 µl). Après 20 minutes d'agitation, la résine est lavée par deux lavages successifs au DMF. On additionne alors à la résine presque sèche 10 eq des réactifs suivants : Fmoc-Gly-OH, HBTU, HOBt et 30 eq de DIEA dans du DMF. La solution est agitée pendant 45 minutes avant d'être lavée au DMF (4x2min) puis au DCM (4x2min). Les autres aminoacides sont ensuite greffés successivement.
- Dérivé du peptide **NS4** fonctionnalisé par un groupe 4,7,10-trioxa-1,13-diamino-tridecanyl-α-oxoaldéhyde du côté C-terminal :

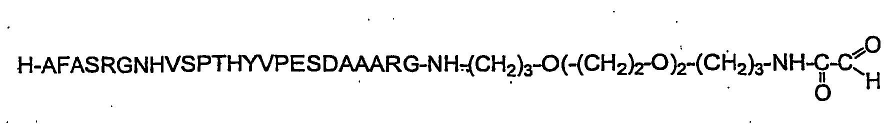

Ce peptide est synthétisé en phase solide depuis l'extrémité C-terminale vers l'extrémité N-terminale selon une stratégie Fmoc sur une résine méthyl-2,3-O-isopropylidène-D-tartryl-Val-PEGA. La synthèse est effectuée selon le même protocole que celle du peptide HCVpc21-2.

### 2 - Peptide du virus EBV

- Dérivé du peptide **VCA p18 (EBV)** fonctionnalisé par un groupe 4,7,10-trioxa-1,13-diamino-tridecanyl-α-oxoaldéhyde du côté C-terminal :

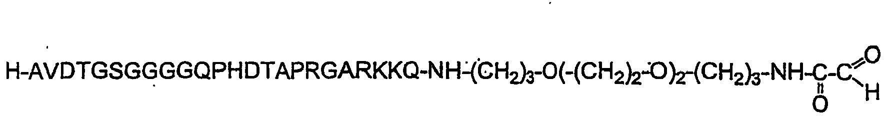

Ce peptide est synthétisé en phase solide depuis l'extrémité C-terminale vers l'extrémité N-terminale selon une stratégie Fmoc sur la résine méthyl-2,3-O-isopropylidène-D-tartryl-val-PEGA suivant le même protocole que pour le peptide HCVpc21-2.

### Exemple 4 - Protocole d'utilisation des lames semicarbazides

### 1 - Ligation des peptides

Les peptides fonctionnalisés sont tout d'abord solubilisés (à 10⁻³ M ou 10⁻⁴ M) dans du tampon acétate 0,1M pH 5,5. Ils sont alors distribués dans les puits d'une plaque ELISA de 384 puits (Microtest TM, Becton Dickinson, NJ USA). A l'aide d'un « Spotteur » manuel à 32 aiguilles (Microarray Printer XMM 47832-Xenopore, Hawthorne, US), les peptides sont prélevés depuis la plaque ELISA et déposés sur une lame de verre semicarbazide.
Les lames sont alors mises à incuber 1 nuit à 37°C sous atmosphère humide.

Ces lames sont ensuite lavées, par passage de 60 minutes sous ultrasons dans une solution de Tris-acétate (0,1M Tris(hydroxyméthyl)-aminométhane -Merck, Darmstadt, Allemagne-).

Les lames sont ensuite lavées 4 fois pendant 3 minutes en solution de PBS (Tampon phosphate 0,01M additionné à 1,8% de NaCl pH 7,4) en présence de 0,05% de Tween 20.

### 2 - Mise en contact des sérums-tests

Les lames sont alors incubées en présence de 100µl de sérum de patients dilué au 1/50^{éme} dans le tampon de dilution (PBS + 2,5% de lait demi-écrémé en poudre + 0,5% de Tween 20) sous une lamelle de verre couvre-objet (24x60mm). L'incubation des lames s'effectue pendant 2 heures à 37°C sous atmosphère humide. On réalise alors 4 lavages successifs pendant 3 minutes en solution de PBS additionné de 0,05% de Tween 20.

### 3 - Détection d'anticorps spécifiques dans les sérums par mesure de la fluorescence

La réaction des anticorps de patients sur les polypeptides fixés sur la lame est détectée par la fixation, sur ceux-ci, d'anticorps anti-Ig humaines fluorescents.

100 µl de solution d'anticorps anti-IgG-A-M humaines marqués à la rhodamine (TRITC) (Jackson ImmunoResearch Laboratories, Baltimore, US) diluée au 1/100^{ème} dans le tampon de dilution (PBS + 2,5% de lait demi-écrémé en poudre + 0,5% de Tween 20) sont ensuite déposés sur chaque lame. On recouvre alors chaque lame par une lamelle (24x60 mm) et on la laisse incuber 1 heure à 37°C sous atmosphère humide. Une série de 4 lavages successifs en solution de PBS additionné de 0,05% de Tween 20 est réalisée. Les lames sont ensuite rincées à l'aide d'une pissette d'eau mQ puis d'éthanol 95° pendant 1 minute, puis séchées 15 minutes à l'air ambiant.

La fluorescence émise est ensuite détectée à l'aide d'un scanner à lame (L35/PMT 50, Affymetrix 418 Array Scanner, MWG).

### Exemple 5 - Utilisation des lames comme « puces » à polypeptides pour la détection d'anticorps présents dans des sérums

### 1 - Etude sur le peptide HCVpc21

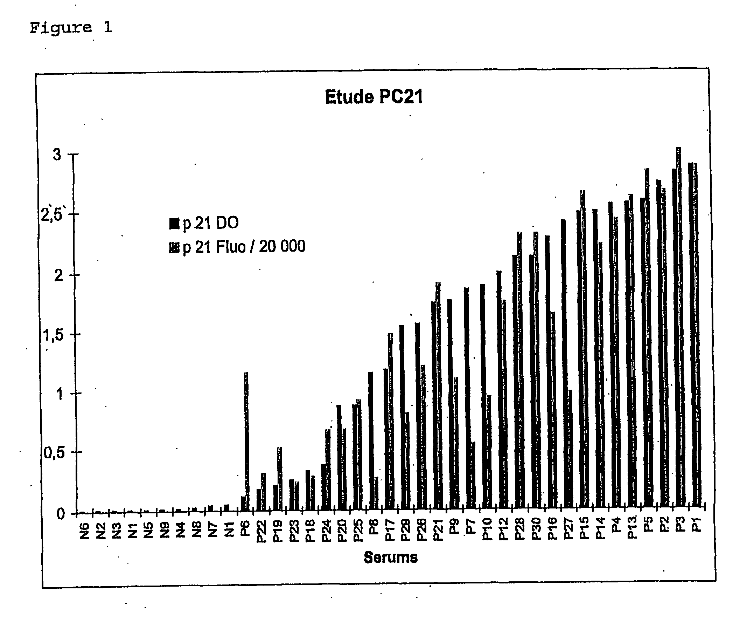

Des sérums de patients dont la sérologie est connue (c'est-à-dire vérifiée avec des tests de référence) ont été comparés en test ELISA et par les « biopuces » selon l'invention :
30 sérums positifs numérotés de P1 à P30
10 sérums négatifs numérotés de N1 à N10
Les lames selon l'invention sont utilisées avec 16 spots du peptide HCVpc21-2 (décrit dans l'exemple 3).
Les tests ELISA sont effectués sur des plaques COSTAR carbobind, avec le même peptide HCVpc21-2.
Les résultats du test ELISA, exprimés en densité optique, et les résultats du test « biopuces » exprimés en fluorescence/2,0000 (moyenne de la mesure de la fluorescence des 16 spots par échantillon) sont présentés sur la figure 1.
Le test biopuces s'est révélé sensible et spécifique à 100% pour cette étude sur ces sérums HCV référencés, puisqu'il a permis de détecter tous les positifs et aucun négatif.

### 2 - Comparaison entre la densité optique obtenue avec le test ELISA et la fluorescence des lames semicarbazides pour le peptide HCVpc21

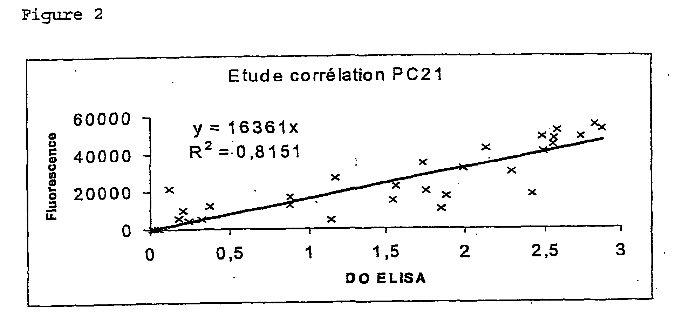

Les résultats de l'étude précédente ont été reportés sur la figure 2 qui montre la corrélation entre fluorescence et densité optique.

On n'observe pour les sérums négatifs aucune fluorescence (fluorescence correspondant au bruit de fond), ce qui n'est pas le cas pour l'ELISA où les négatifs ont une valeur. Dès qu'une fluorescence est observée avec la technique biopuce on sait que c'est un positif, ce qui n'est pas le cas en ELISA où une faible valeur de DO peut correspondre à un négatif. Ceci implique en ELISA la définition d'une valeur seuil qui correspond à la moyenne des sérums négatifs + 3 fois leur écart type ; avec les biopuces, la valeur seuil est la moyenne du bruit de fond + 3 fois l'écart type, ce qui correspond à une valeur très faible contrairement à l'ELISA. Par conséquent, le différentiel entre sérums négatifs et positifs est beaucoup plus important pour les biopuces que pour l'ELISA.

De plus il est encore possible d'augmenter le gain de sensibilité en augmentant la puissance du scanner. Ainsi on garde toujours une valeur de fluorescence correspondant au bruit de fond pour les sérums négatifs, par contre on augmente la valeur de fluorescence des spots positifs ; ceci est particulièrement intéressant dans le cas de sérums faiblement positifs que l'on détecte alors sans aucune ambiguité.

### 3 - Etude de reproductibilité

Le tableau suivant exprime la fluorescence moyenne observée sur 9 lames différentes, vis à vis d'un même sérum identifié HCV positif par le test ELISA. Sur chaque lame ont été réalisés 120 spots du peptide HCVpc21-2 " grand linker " à la concentration de 10⁻⁴ M.
La moyenne de fluorescence observée sur les 9 lames est de 41095.
L'écart type sur les 9 lames est de 1891, soit un écart type de 4,5%.

**Tableau I**

| Lames N° | Fluorescence |
|---|---|
| L1 | 42104 |
| L2 | 40282 |
| L3 | 37676 |
| L4 | 40853 |
| L5 | 40337 |
| L6 | 42375 |
| L7 | 39568 |
| L8 | 43918 |
| L9 | 42734 |

### 4 - Comparaison entre les différents types de peptides HCVpc21

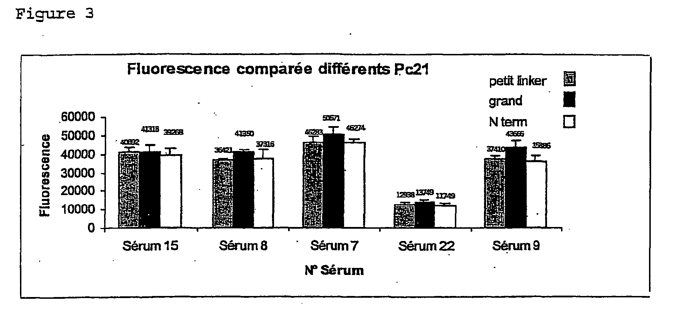

Cette étude a été réalisée sur 5 lames différentes (5 sérums différents, 1 sérum par lame, sérums HCV positifs par le test ELISA carbobind HCVpc21 et par le test EIA 3.0 de Abbott, actuellement sur le marché).
Sur chaque lame, 8 spots par peptide HCVPc21 ont été déposés à la concentration de 10⁻⁴ M. 3 types de peptides différents (tels que décrits dans l'exemple 3) ont été étudiés, HCVpc21-1, HCVpc21-2, HCVpc21-3. Les résultats sont présentés sur la figure 3.

La fluorescence est plus élevée avec le peptide HCV " grand linker ". Les résultats sont comparables pour le peptide " petit linker " et HCVpc21-1. Le gain de fluorescence moyen sur les 5 lames est de 9.9% en faveur du " grand linker " par rapport au " petit linker " et est de 11.11% pour le " grand linker " par rapport au peptide HCVpc21-1 (fonction COCHO sur la position N-terminale).

### 5 - Etude de sérums avec les peptides HCVpc21, NS4, et un mélange HCVpc21/NS4

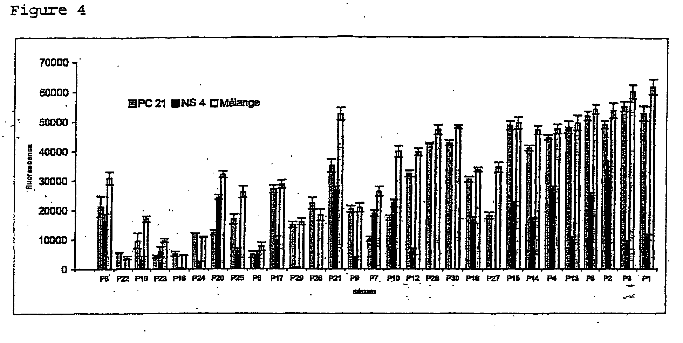

Le peptide HCVpc21 est « spotté » selon le protocole décrit dans l'exemple 4 à la concentration de 10⁻⁴ M, le peptide NS4 est spotté à 10⁻³ M. Pour le mélange, le peptide NS4 est à une concentration de 0,5 10⁻³M et le peptide HCVpc21 à une concentration de 0,5 10⁻⁴M (ratio 10/1). Chaque peptide est spotté 16 fois par lame. Les résultats exprimés correspondent à la fluorescence moyenne des 16 spots.
Les résultats présentés sur la figure 4 concernent 30 sérums référencés HCV positifs selon le test de la société Abbott. Ces sérums ont été numérotés de P1 à P30.
Le test selon l'invention a permis la détection de tous les sérums positifs. 10 sérums témoins négatifs ont été testés, pour lesquels aucune fluorescence n'a été détectée (fluorescence correspondant au bruit de fond).
Le test s'est donc révélé sensible et spécifique à 100% sur les échantillons testés.

### 6 - Essai sur le sang total pour le virus EBV

Sur une lame de verre semicarbazide, 16 spots du peptide EBV (10⁻⁴ M) et 16 spots du peptide HCV (10⁻⁴ M) ont été réalisés.
Un échantillon de sang frais (patient négatif pour HCV et positif pour EBV), de 100 µl prélevés au bout du doigt a été dilué dans 50 µl de tampon de dilution .(tampon PBS, Tween 0,1%, lait demi-écrémé 2,5%). Ces 150 µl sont alors déposés sur la lame et mis à incuber 2 heures à 37°C sous une lamelle.
Le protocole de révélation des lames est le même que précédemment décrit.

Résultats : La fluorescence HCV est nulle, la fluorescence EBV est de 22991 (moyenne des 16 spots, écart type de 8,1% sur ces 16 spots).

### 7 - Mise en évidence de la sensibilité des biopuces à polypeptides

Pour une puissance de scanner L35/PMT50, le bruit de fond après la mise en contact avec le sérum et l'anticorps fluorescent est en moyenne de 50, à savoir 0.1% du signal maximum observé (étude statistique sur 100 sérums référencés). Ce bruit de fond est 16 fois inférieur à celui obtenu sur plaques Carbobind^{®} avec les mêmes peptides.
D'autre part, pour les sérums négatifs, aucune fluorescence n'est observée au niveau des spots de peptides, hormis celle attribuée au bruit de fond.

### Exemple 6 - Utilisation d'un peptide fonctionnalisé par un groupement cétone

Un peptide dérivé du. HCVpc21 a été synthétisé en phase solide depuis l'extrémité N-terminale selon une stratégie Fmoc/tBu, sur une résine Fmoc-PAL-PEG-PS.
Une partie du produit (HCVpc21-Met) sera utilisé comme contrôle :
H-MTNRRPQDVKFPGGGQIVGGVYLLPRRGPRLG-NH₂

Une partie du produit a subi une transamination pour donner le dérivé suivant (HCVpc21-TA) :

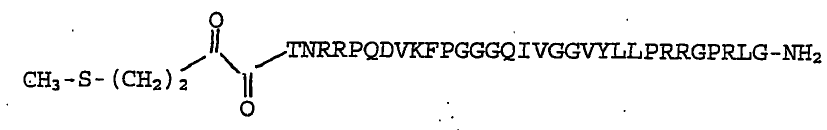

La réaction est effectuée selon le protocole suivant :
Une partie du produit précédent (m=10,45 mg) est solubilisée dans 1165 µl d'eau ; on ajoute à cette solution 1,165 ml d'une solution aqueuse à pH 5,5 contenant : acétate de sodium 2M, acide acétique 0,2M, sulfate de cuivre 2,5 mM, acide glyoxylique 0,1M. Après 5 heures de réaction, on additionne 1,165 ml d'EDTA 7mM sous agitation. La solution obtenue est purifiée par HPLC préparative et les fractions correspondant au produit sont lyophilisées.

Les 2 peptides HCVpc21-Met (non transaminé) et pc21-TA (transaminé) ainsi que le peptide HCVpc21-2 (décrit dans l'exemple 3) ont été fixés sur des lames semicarbazides à raison de 16 spots de chaque peptide par lame.
Chaque peptide est déposé à la concentration de 10⁻⁴M.
Les lames sont incubées en présence de 4 sérums positifs et de 2 sérums négatifs. Les résultats présentés dans le tableau suivant montrent que
* le peptide transaminé se lie correctement à la lame semicarbazide,
* le peptide contrôle pc21-Met donne une réponse faible correspondant à une absorption non covalente (non spécifique).

**Tableau II**

| Fluorescence Moyenne + (E.T. %) | | | | |
|---|---|---|---|---|
| Peptides : | pc21-2 | pc21-TA | pc21-Met | Bruit de fond |
| Sérum 43 | 18158 | 12910 | 2810 | 50 |
| DO/ELISA 1,7 | (5, 9) | (6,3) | (10,2) | |
| Sérum 45 | 968 | 1880 | 356 | 58 |
| DO/ELISA 0,7 | (6,2) | (10,1) | (5,6) | |
| Sérum 53 | 26963 | 23063 | 7741 | 56 |
| DO/ELISA 2,7 | (7,1) | (6, 8) | (7,8) | |
| Sérum 41 | 6225 | 6169 | 960 | 61 |
| DO/ELISA 1,4 | (5, 5) | (6,5) | (5,0) | |
| Sérum 48 | 58 | 58 | 59 | 57 |
| Négatif | | | | |
| Sérum 55 | 56 | 54 | 55 | 54 |
| Négatif | | | | |

### Exemple 7 - Fixation d'une protéine glycosylée sur les lames semicarbazides

### (Exemple comparatif)

A titre de modèle de fixation d'une protéine glycosylée sur les lames semicarbazides, on utilise un anticorps fluorescent, ce qui permet sa détection directe.
Un anticorps anti-IgG-A-M rhodaminé, est oxydé pour générer une fonction aldéhyde selon le mode opératoire décrit par Wolfe et Hage (Anal. Bioch. ; 231 ; 123-130 1995) :
440 µl d'anticorps (1,5 mg/ml), dilués dans un tampon 0,02M acétate de sodium 0,15M NaCl à pH 5,5 sont placés à 4°C pendant 15 minutes. Une solution de periodate de sodium 20mM dans le même tampon est également placée à 4°C à l'abri de la lumière. 275 µl de la solution de periodate de sodium sont additionnés à 275 µl de la solution d'anticorps à l'abri de la.lumière, sous agitation : solution A.
275 µl de la solution de tampon 0,1M acétate de sodium 0,15M NaCl à pH 5,5 sont additionnés à 275 µl de la solution d'anticorps à l'abri de la lumière, sous agitation : solution B.
Les deux solutions sont placées à 4°C pendant 20 minutes. On additionne alors à chacune d'elles 137 µl d'éthylène glycol (0,25 ml par millilitre d'échantillon) sous agitation.
Ces deux solutions sont alors transvasées dans un tube à dialyse (Nanosep, Microconcentrators, GelmanSciences, Filtron Brand).
L'excès de réactif est éliminé par une série de 4 centrifugations effectuées sans sécher la membrane (3 000 tours par minute, 60 minutes puis 10 000 tours par minute, 20 minutes) en rediluant chaque fois dans la solution de tampon acétate à pH 5,5 (ajout de 200 µl de tampon).
Ces deux solutions ont été prélevées à l'aide d'un minicaps de 1 µl (Hirschmann Laborgerate) et spottées 8 fois sur une lame de verre fonctionnalisé par des groupes semicarbazides comme écrit précédemment.
La lame est ensuite mise à incuber 6 heures à 37°C sous atmosphère humide. Elle est alors lavée par trempage dans une solution de Tris acétate 0,1 M Tween 20 0,1% pendant 60 minutes sous ultra-sons. La lame est ensuite rincée à l'eau et séchée à l'éthanol. La fluorescence est révélée au scanner à lames.

Les valeurs de fluorescence obtenues pour l'anticorps oxydé (solution A) et l'anticorps non oxydé (solution B) sont présentées dans le tableau suivant :

| | Fluorescence | Ecart type (%) |
|---|---|---|
| Anticorps oxydé | 26 270 | 9,8 |
| Anticorps non oxydé | 6 068 | 14,5 |
| Bruit de fond | 141 | |

### Exemple 8, criblage épitopique.

### Synthèse :

Une chimiothèque de 24 décapeptides a été synthétisée en parallèle afin de couvrir 3 boucles exposées au solvant de la protéine NS3. La synthèse a été réalisée en phase solide depuis l'extrémité C-terminale vers l'extrémité N-terminale selon la stratégie classique Fmoc/ *ter-*butyle et une activation in situ PyBOP/DIEA sur la résine "Gly-4, 7,10-trioxa-1,13-diamino-tridecanyl-méthyl-2', 3'-O-isopryldène-D-tartryl-Val-PEGA", préparée comme décrit pour le peptide HCVpc21-2.

La synthèse des 24 peptides a été effectuée sur un synthétiseur automatique multiple de 96 canaux de type ACT 496 MOS de Advanced Chemtech. Les synthèses ont été entreprises sur une échelle de 0,02 mmole et 15 eq. d'acide activé ont été utilisés pour chaque étape de couplage (simples couplages). Les peptidyl-résines ont été acétylées avec le mélange Ac₂O/DIEA/NMP (3/0,3/86,7) après chaque couplage. En fin de synthèse, les résines ont été transférées dans des seringues munies de frittés (de marque ABIMED) et déprotégées à température ambiante avec 1 ml du mélange TFA/H₂O/éthanedithiol (95/2,5/2,5 par vol.) pendant 2 heures. Les résines ont ensuite été lavées avec du CH₂Cl₂, du méthanol et finalement de l'éther éthylique et séchées. Les résines ont été conditionnées dans une solution d'acide acétique à 10% dans l'eau, puis traitées par 6 eq. de NaIO₄(25,7 mg) dissous dans 500 µl d'eau pendant 2 minutes. La coupure oxydante a été stoppée par ajout de 24 équivalents d'éthanolamine (29 µl). Après deux lavages par 1 ml d'eau, les filtrats ont été mélangés et dessalés par RP-HPLC (colonne C3-Zorbax, 0% B à 100% B, débit 5 ml.min⁻¹, détection 230 nm) . Après lyophilisation, les peptides ont été analysés par RP-HPLC analytique et par spectrométrie de masse MALDI-TOF. Les résultats obtenus sont rassemblés dans le tableau suivant.
La formule générique des peptides est indiquée dans la figure ci-dessous.

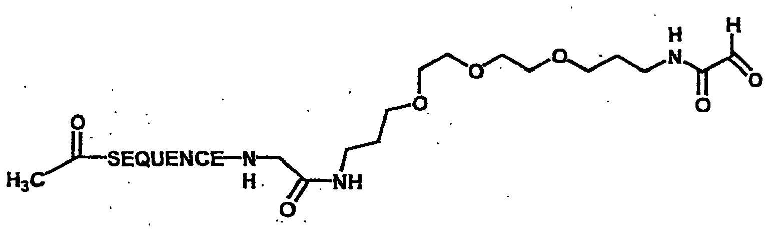

| *Peptide* | *Séquences* | *Rdt %* | *Pureté HPLC* | *[M+H]⁺ calc.* | *m*/*z observé Pic Principal* |
|---|---|---|---|---|---|
| **1** | **YGKAIPLEAI** | **30** | **89%** | **1431,67** | **1453,64 (+Na)** |
| **2** | **GKAIPLEAIK** | **32** | **90%** | **1396,67** | **1418,75 (+Na)** |
| **3** | **KAIPLEAIKG** | **30** | **91%** | **1396,67** | **1418,77 (+Na)** |
| **4** | **AIPLEAIKGG** | **30** | **80%** | **1325,54** | **1347,70 (+Na)** |
| **5** | **IFLEAIKGGR** | **30** | **76%** | **1410,65** | **1410,99** |
| **6** | **PLEAIKGGRH** | **35** | **81%** | **1434,63** | **1435,98 (+H)** |
| **7** | **LEAIKGGRHL** | **31** | **76%** | **1450,68** | **1451,02** |
| **8** | **EAIKGGRHLI** | **34** | **75%** | **1450,68** | **1451,98 (+H)** |
| **9** | **ELAAKLSGLG** | **32** | **72 %** | **1315,50** | **1337,86 (+Na)** |
| **10** | **LAAKLSGLGI** | **27** | **75%** | **1299,55** | **1321,91 (+Na)** |
| **11** | **AAKLSGLGIN** | **30** | **72 %** | **1300,49** | **1322,87 (+Na)** |
| **12** | **AKLSGLGINA** | **29** | **72 %** | **1300,49** | **1322,86 (+Na)** |
| **13** | **KLSGLGINAV** | **23** | **72%** | **1328,55** | **1350,87 (+Na)** |
| **14** | **LSGLGINAVA** | **29** | **65%** | **1271,45** | **1293,88 (+Na)** |
| **15** | **SGLGINAVAY** | **60** | **86 %** | **1321,47** | **1343,83 (+Na)** |
| **16** | **GLGINAVAYY** | **6** | **15%** | **1397,57** | **1419,90 (+Na)** |
| **17** | **GLDVSVIPTS** | **29** | **36 %** | **1344,50** | **1366,84 (+Na)** |
| **18** | **LDVSVIPTSG** | **28** | **78%** | **1344,5** | **1366,85 (+Na)** |
| **19** | **DVSVIPTSGD** | **26** | **63%** | **1346,43** | **1368,80 (+Na)** |
| **20** | **VSVIPTSGDV** | **28** | **60%** | **1330,47** | **1352,77 (+Na)** |
| **21** | **SVIPTSGDVV** | **30** | **64%** | **1330,47** | **1352,67 (+Na)** |
| **22** | **VIPTSGDVVV** | **26** | **86 %** | **1342,53** | **1364,70 (+Na)** |
| **23** | **IPTSGDVVVV** | **21** | **40 %** | **1342,53** | **1364,68 (+Na)** |
| **24** | **PTSGDVVVVA** | **17** | **46%** | **1300,45** | **1322,65 (+Na)** |

### Test sur biopuce :

Ces peptides dérivés de la protéine NS3 de HCV ont été imprimés sur lame de verre semicarbazide et testés avec 4 sérums référencés NS3+ (Recombinant ImmunoBlot Assay, DECISCAN HCV PLUS) et un sérum référencé HCV- avec le même test comme indiqué dans l'exemple 4 (Protocole d'utilisation des lames semicarbazide).

Cette expérience permet d'identifier, dans la collection de 24 peptides, ceux qui sont les plus intéressants pour la détection d'anticorps dirigés contre NS3.
Les résultats de sérodétection rassemblés dans le tableau ci-dessous montrent que les peptides 14 et 15 sont intéressants pour la détection d'anticorps anti-NS3.,'et que de manière générale, les biopuces à peptides constituent un outil intéressant pour le screening d'épitopes.

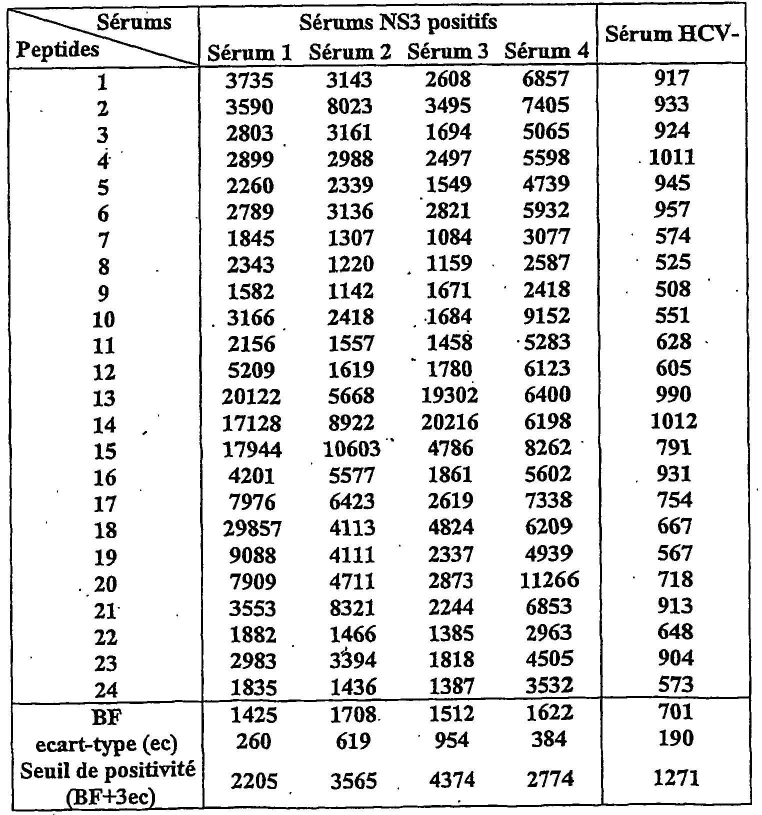

### Exemple 9, - étude de relations ligand-récepteur : le ligand est un peptide biotinylé et le récepteur est la streptavidine ou un anticorps anti-biotine.

### Synthèse du peptide

H-Lys (Biotine-G₂) AYVLAG-NH (CH₂)₃O(CH₂)₂O (CH₂)₂O (CH₂) ₃NHCOCHO

La synthèse a été effectuée sur résine Novasyn TGR (Novabiochem, lot A 23633, charge 0.2 mmol/g) sur une échelle de 0,1 mmole.
Cette résine a été traitée comme décrit pour le peptide HCVpc21-2, la résine PEGA étant remplacée par la résine Novasyn TGR. Après le couplage de la Fmoc-Gly-OH, la synthèse a été poursuivie sur synthétiseur Pionneer de Perseptive'Biosystems, en utilisant la stratégie Fmoc/tert-butyle. Les acides suivants ont été couplés successivement (10 éq., simple couplage, activation TBTU/HOBt/DIEA) : Fmoc-L-Ala-OH, Fmoc-L-Leu-OH, Fmoc-L-Val-OH, Fmoc-L-Tyr(OtBu)-OH, Fmoc-L-Ala-OH, Boc-L-Lys(Fmoc)-OH, Fmoc-Gly-OH deux fois, biotine.
La déprotection et la coupure du peptide de la résine a été effectuée avec 10 ml du mélange TFA95/H₂0 2,5/diméthylsulfure 2,5 (1h à température ambiante). Le peptide a été précipité dans l'éther éthylique/pentane . 1/1 (par vol.), centrifugé, repris dans AcOH à 30% dans l'eau et lyophilisé (69 mg). Le peptide est purifié sur colonne C18 Nucleosil (A : H₂O 0,05% TFA, B : CH₃CN/H₂O : 4/1 en vol . 0,05% TFA 0-25% 20 min, 25-25% 5 min, 25-35% 40 min.). On obtient 35,72 mg de produit pur.

L'oxydation est réalisée avec 6 éq. de NaIO₄ et 8 éq. de méthionine à une concentration de 0,5 mM dans phosphate 100 mM pH 6,6/MeOH : 1/1 en vol pendant 15 min. La réaction est arrêtée avec 12 éq. d'éthanolamine, diluée à l'eau et purifiée sur colonne C18 Nucléosil (0-23% 20 min, 23-23% 5 min, 23-35%, 35 min) pour donner le produit attendu.

Le peptide pep-biotine est "spotté" selon le protocole décrit dans l'exemple 4 paragraphe 1 aux concentrations de 10⁻³M, 10⁻⁴M, 10⁻⁵M et 10⁻⁶M. Chaque concentration est spottée 6 fois par lame.
Les lames sont ensuite incubées en présence de 150 µl de ligand (solution de Rhodamine Conjugated affinity Purified anti-Biotin [goat] (Rockland, Gilbertsville, PA, USA) ou solution de Streptavidin Tetramethyl Rhodamine conjugate (Molecular Probes, Oregon, USA)) dilué dans un tampon PBS 0,01 M, pH 7,2 (concentrations de ligand : 10⁻¹mg/ml, 10⁻²mg/ml 10⁻³mg/ml, 10⁻⁴mg/ml) sous une lamelle de verre couvre-objet (24x60mm) . L'incubation des lames s'effectue pendant 2 heures à 37°C sous atmosphère humide. Une série de 4 lavages successifs en solution de PBS additionné de 0,05% de Tween 20 est réalisée. Les lames sont ensuite rincées à l'aide d'une pissette d'eau desionisée puis d'éthanol à 95° pendant 1 minute, puis séchées 15 minutes à l'air ambiant.
La fluorescence est ensuite détectée à l'aide d'un scanner à lame (L35/PMT50, Affymetrix 418 Array Scanner, MWG). Les résultats exprimés dans le tableau suivant correspondent à la fluorescence moyenne et à l'écart type des 6 spots de chaque concentration obtenus après incubation de la streptavidine rhodaminée et de l'anticorps anti-biotine rhodaminé respectivement.

| **[streptavidine rhodaminée]** | | | | | | | | |
|---|---|---|---|---|---|---|---|---|
| | **10⁻¹ mg/ml** | | **10⁻² mg/ml** | | **10⁻³ mg/ml** | | **10⁻⁴ mg/ml** | |
| **[pep-Biotine]** | **Moyenne** | **Ecart type** | **Moyenne** | **Ecart type** | **Moyenne** | **Ecart type** | **Moyenne** | **Ecart type** |
| **10⁻³ mg/ml** | **34660** | **2540** | **nd** | **nd** | **29836** | **1515** | **13816** | **4039** |
| **10⁻⁴ mg/ml** | **31781** | **3056** | **nd** | **nd** | **24666** | **2886** | **7931** | **1435** |
| **10⁻⁵ mg/ml** | **26524** | **2095** | **nd** | **nd** | **20902** | **2554** | **7673** | **818** |
| **10⁻⁶ mg/ml** | **18500** | **1916** | **nd** | **nd** | **10737** | **887** | **2124** | **733** |
| **Bruit de fond** | **10062** | **515** | **nd** | **nd** | **580** | **337** | **174** | **36** |

| **[Ac anti-biotine rhodaminée]** | | | | | | | | |
|---|---|---|---|---|---|---|---|---|
| | **10⁻¹ mg/ml** | | **10⁻² mg/ml** | | **10⁻³ mg/ml** | | **10⁻⁴ mg/ml** | |
| **[pep-Biotine]** | **Moyenne** | **Ecart type** | **Moyenne** | **Ecart type** | **Moyenne** | **Ecart type** | **Moyenne** | **Ecart type** |
| **10⁻³ mg/ml** | **15350** | **1172** | **13113** | **637** | **6828** | **883** | **2271** | **348** |
| **10⁻⁴ mg/ml** | **14721** | **1443** | **9121** | **1974** | **4981** | **1065** | **1535** | **511** |
| **10⁻⁵ mg/ml** | **10932** | **1475** | **6849** | **933** | **3377** | **292** | **1249** | **67** |
| **10⁻⁶ mg/ml** | **6606** | **125** | **2947** | **825** | **791** | **87** | **385** | **39** |
| **Bruit de fond** | **1424** | **109** | **243** | **13** | **167** | **19** | **170** | **12** |

Etude de compétition sur la biotine montrant la spécificité de l'interaction ligand récepteur :

Le peptide pep-biotine est "spotté" selon le protocole décrit dans l'exemple 4 paragraphe 1 aux concentrations de 10-³ M, 10⁻⁴M, 10⁻⁵M et 10⁻⁶M. Chaque concentration est spottée 6 fois par lames.
Les lames sont ensuite incubées en présence de 150 µl d'une solution à 10⁻³ mg/ml de streptavidine rhodaminée (solution de streptavidin Tetramethyl Rhodamine conjugate (Molecular Probes, Oregon, USA)) diluée dans un tampon PBS 0,01 M, pH 7,2 en présence de différentes concentrations de biotine (10⁻³ M, 10⁻⁴M, 10⁻⁵M, 10⁻⁷M et 0M) sous .une lamelle de verre couvre-objet (24x60mm). L'incubation des lames s'effectue pendant 2 heures À 37°C sous atmosphère humide. Une série de 4 lavages successifs en solution de PBS additionné de 0,05% de Tween 20 est réalisée. Les lames sont ensuite rincées, séchées et analysées comme indiqué précédemment. Les résultats exprimés dans le tableau suivant correspondent à la fluorescence moyenne et à l'écart type des 6 spots de chaque concentration obtenus après incubation.

| **[Biotine] en compétition** | | | | | | | | | | |
|---|---|---|---|---|---|---|---|---|---|---|
| | **0M** | | **10⁻⁷ M** | | **10⁻⁵ M** | | **10⁻⁴ M** | | **10⁻³ M** | |
| | **M** | **ET** | **M** | **ET** | **M** | **ET** | **M** | **ET** | **M** | **ET** |
| **[pep-Biotine]** | | | | | | | | | | |
| **10⁻³ mg/ml** | **29836** | **1515** | **17132** | **7141** | **16683** | **1615** | **2237** | **394** | **376** | **78** |
| **10⁻⁴ mg/ml** | **24666** | **2886** | **12178** | **406T** | **9414** | **686** | **961** | **65** | **207** | **25** |
| **10⁻⁵ mg/ml** | **20902** | **2554** | **6129** | **1701** | **3588** | **840** | **322** | **42** | **107** | **10** |
| **10⁻⁶ mg/ml** | **10737** | **887** | **2611** | **441** | **2350** | **496** | **189** | **8** | **78** | **13** |
| **Bruit de fond** | **580** | **337** | **116** | **15** | **146** | **8** | **95** | **7** | **75** | **6** |

| | | | | | | | | | | |
|---|---|---|---|---|---|---|---|---|---|---|
| M: moyenne de fluorescence ET : écart-type sur la fluorescence | | | | | | | | | | |

## Revendications

1. Dispositif de présentation de polypeptides, **caractérisé en ce qu'**il consiste en un support plat sur lequel lesdits polypeptides sont liés de manière covalente, **caractérisé en ce que** la liaison entre les polypeptides et le support résulte de la formation d'un lien semicarbazone qui résulte de la réaction entre
- des polypeptides portant une fonction aldéhyde ou cétone
- et un support fonctionnalisé par des groupements semi-carbazides , et **en ce que** les fonctions aldéhyde ou cétone portées par les polypeptides sont des α-oxoaldéhyde ou α-oxocétone, et sont situées du côté C-terminal ou du côté N-terminal, ou sur une chaîne latérale.

2. Dispositif selon la revendication 1, **caractérisé en ce que** le support est constitué en un matériau solide, organique ou inorganique, de type verre, silicium, polymères synthétiques, et présentant une surface plane.

3. Dispositif selon la revendication 1 ou 2, **caractérisé en ce que** les sites qui, sur ledit support, se trouvent entre lesdits polypeptides, sont liés de manière covalente à un PEG dérivatisé par une fonction alpha-oxoaldéhyde ou alpha-oxocétone, via le même lien semicarbazone que celui qui est utilisé pour immobiliser lesdits polypeptides.

4. Dispositif selon l'une quelconque des revendications 1 à 3, **caractérisé en ce que** la qualité de la fonctionnalisation du support (densité et homogénéité) est contrôlée par sa capacité de fixer une sonde peptidique de synthèse, fluorescente, dérivatisée par une fonction α-oxoaldéhyde.

5. Dispositif selon l'une quelconque des revendications 1 à 4, **caractérisé en ce que** ladite fonction alpha-oxoaldéhyde ou alpha-oxocétone est située sur un bras espaceur.

6. Procédé de préparation d'un dispositif selon l'une quelconque des revendications 1 à 5, **caractérisé en ce qu'**il comprend les étapes suivantes :
1- introduction d'une fonction alpha-oxoaldéhyde ou alpha-oxocétone, par synthèse ou par modification d'une fonction naturelle, à l'une des extrémités N ou C ou sur une chaîne latérale d'un polypeptide de synthèse ou naturel ;
2- fonctionnalisation d'un support solide par des groupements semicarbazides ;
3- dépôt sous forme de spots d'échantillons de polypeptides obtenus par l'étape 1, sur le support fonctionnalisé de l'étape 2, dans des conditions de pH et d'humidité assurant la réaction entre la fonction alpha-oxoaldéhyde ou alpha-oxocétone et la fonction semicarbazide pour créer le lien semicarbazone.

7. Procédé selon la revendication 6, **caractérisé en ce que** l'étape 1 est effectuée en cours de synthèse d'un polypeptide à l'aide d'un synthétiseur automatique.

8. Procédé selon la revendication 6 ou 7, **caractérisé en ce que** l'étape 1 comprend en outre l'introduction d'un bras espaceur entre le dernier acide aminé de la séquence du polypeptide et la fonction alpha-oxoaldéhyde ou alpha-oxocétone.

9. Procédé selon la revendication 6, **caractérisé en ce que** l'étape 1 est effectuée par transamination d'un acide aminé N-terminal d'une protéine naturelle, glycosylée ou non, ou d'un fragment de celle-ci.

10. Procédé selon l'une quelconque des revendications 6 à 9, caractérisé en en ce que l'étape 2 comprend
- une réaction de silanisation du support, introduisant une fonction amine ;
- la transformation de la fonction amine en fonction isocyanate ;
- la réaction de la fonction isocyanate avec un dérivé d'hydrazine pour former le groupement semicarbazide.

11. Procédé selon l'une quelconque des revendications 6 à 9, caractérisé en en ce que l'étape 2 est effectuée en une seule réaction d'un silane portant un groupement semicarbazide.

12. Procédé selon la revendication 11, **caractérisé en ce que** le groupement semicarbazide est de préférence protégé par le Fmoc.

13. Procédé selon l'une quelconque des revendications 6 à 12, caractérisé en en ce que l'étape 3 comprend
- la préparation de solutions des polypeptides de l'étape 1 à 10⁻³ ou 10⁻⁴M dans un tampon acétate 0,1 M à pH 5,5,
- leur distribution dans un récipient approprié à leur prélèvement, de type puits de plaque de microtitration,
- leur prélèvement à l'aide d'un « spotteur »,
- et leur dépôt sur le support semicarbazide ;
- l'incubation des lames pendant une nuit à 37°C sous atmosphère humide
- et leur lavage et la saturation des sites réactifs non spécifiques.

14. Utilisation de dispositifs selon l'une quelconque des revendications 1 à 5 comme puces à polypeptides en tant qu'outil de diagnostic.

15. Utilisation de dispositifs selon la revendication 14, **caractérisée en ce qu'**elle comprend la détection des réponses de type antigène-anticorps par l'utilisation *in vitro* de réactifs marqués, fluorescents, radioactifs ou marqués chimiquement, comme dans les tests de diagnostic non miniaturisés.

16. Utilisation de dispositifs selon l'une quelconque des revendications 1 à 5 comme puces à polypeptides pour le criblage de molécules.

17. Utilisation de dispositifs selon l'une quelconque des revendications 1 à 5 comme puces à polypeptides pour l'analyse des relations entre molécules, de type ligand-récepteur.

18. Utilisation d'un dispositif selon l'une quelconque des revendications 1 à 5, comme puce à polypeptides pour la détection miniaturisée de molécules structurellement ou fonctionnellement complémentaires.

## Claims

1. A device for presenting polypeptides, **characterized in that** it consists of a flat support onto which said polypeptides are bonded in a covalent manner, **characterized in that** the bonding between the polypeptides and the support results from the formation of a semicarbazone bond that results from the reaction between:
• polypeptides carrying an aldehyde or ketone function;
• and a support functionalized with semicarbazide groups; and **in that** the aldehyde or ketone functions carried by the polypeptides are α-oxoaldehyde or α-oxoketone functions, and are located on the C-terminal side or on the N-terminal side or on a side chain.

2. The device according to claim 1, **characterized in that** the support is constituted by a solid material, organic or inorganic, of the glass, silicon or synthetic polymer type, and has a flat surface.

3. The device according to claim 1 or claim 2, **characterized in that** on said support, the sites that are located between said polypeptides are covalently bonded to a PEG derivatized by an alpha-oxoaldehyde or alpha-oxoketone function via the same semicarbazone bond as that used to immobilize said polypeptides.

4. The device according to any one of claims 1 to 3, **characterized in that** the quality of the functionalization of the support (density and homogeneity) is checked by its capacity to bind a synthetic fluorescent peptide probe derivatized with an α-oxoaldehyde function.

5. The device according to any one of claims 1 to 4, **characterized in that** said alpha-oxoaldehyde or alpha-oxoketone function is located on a spacer arm.

6. A method for preparing a device according to any one of claims 1 to 5, **characterized in that** it comprises the following steps:
1- introducing an alpha-oxoaldehyde or alpha-oxoketone function, by synthesis or by modification of a natural function, at one of the ends, N or C, or onto a side chain of a synthetic or natural polypeptide;
2- functionalizing a solid support with semicarbazide groups;
3- depositing, in the form of spots, samples of polypeptides obtained in step 1 onto the support that has been functionalized in step 2, under pH and humidity conditions that ensure reaction between the alpha-oxoaldehyde or alpha-oxoketone function and the semicarbazide function in order to create the semicarbazone bond.

7. A method according to claim 6, **characterized in that** step 1 is carried out during the synthesis of a polypeptide with the aid of an automatic synthesizer.

8. A method according to claim 6 or claim 7, **characterized in that** step 1 further comprises introducing a spacer arm between the last amino acid of the polypeptide sequence and the alpha-oxoaldehyde or alpha-oxoketone function.

9. A method according to claim 6, **characterized in that** step 1 is carried out by transamination of an N-terminal amino acid of a natural protein, which may or may not be glycosylated, or of a fragment thereof.

10. A method according to any one of claims 6 to 9, **characterized in that** step 2 comprises:
• a reaction for silanization of the support, introducing an amine function;
• transforming the amine function into an isocyanate function;
• reacting the isocyanate function with a hydrazine derivative to form the semicarbazide group.

11. A method according to any one of claims 6 to 9, **characterized in that** step 2 is carried out in a single reaction of a silane carrying a semicarbazide group.

12. A method according to claim 11, **characterized in that** the semicarbazide group is preferably protected with Fmoc.

13. A method according to any one of claims 6 to 12, **characterized in that** step 3 comprises:
• preparing solutions of polypeptides of step 1 in concentrations of 10⁻³ or 10⁻⁴ in a 0.1 M acetate buffer at a pH of 5.5;
• distributing them into a receptacle which is appropriate for sampling them, of the microtitration well plate type;
• removing them using a spotter;
• and depositing them on the semicarbazide support;
• incubating the plates overnight at 37°C in a moist atmosphere;
• and washing them and saturating the non-specific reactive sites.

14. Use of devices according to any one of claims 1 to 5, as polypeptide chips for a diagnostic tool.

15. Use of devices according to claim 14, **characterized in that** it comprises detecting antigen-antibody type responses by using labeled, fluorescent, radioactive or chemically labeled reagents as in non-miniaturized diagnostic tests.

16. Use of devices according to any one of claims 1 to 5, as polypeptide chips in order to screen molecules.

17. Use of devices according to any one of claims 1 to 5 as polypeptide chips for the analysis of relationships of the ligand-receptor type between molecules.

18. Use of a device according to any one of claims 1 to 5, as a polypeptide chip for the miniaturized detection of structurally or functionally complementary molecules.

## Patentansprüche

1. Vorrichtung zur Darstellung von Polypeptiden, **dadurch gekennzeichnet, dass** sie aus einer Trägerplatte besteht, auf welcher die Polypeptide kovalent gebunden sind, **dadurch gekennzeichnet, dass** die Bindung zwischen den Polypeptiden und dem Träger aus der Bildung einer Semicarbazonbindung resultiert, welche aus der Reaktion zwischen
- Polypeptiden mit einer Aldehyd- oder Ketonfunktion
- und einem Träger resultiert, welcher durch Semicarbazidgruppen funktionalisiert ist, und dadurch, dass die durch die Polypeptide getragenen Aldehyd- oder Ketonfunktionen α-Oxoaldehyde oder α-Oxoketone sind und sich am C-terminalen oder am N-terminalen Ende oder an einer Seitenkette befinden.

2. Vorrichtung nach Anspruch 1, **dadurch gekennzeichnet, dass** der Träger aus einem festen organischen oder anorganischen Material besteht, vom Typ Glas, Silicium, synthetischen Polymeren, und eine plane Oberfläche aufweist.

3. Vorrichtung nach den Ansprüchen 1 oder 2, **dadurch gekennzeichnet, dass** die Stellen auf dem Träger, welche sich zwischen den Polypeptiden befinden, kovalent an ein durch eine α-Oxoaldehyd- oder α-Oxoketonfunktion derivatisiertes PEG über die gleiche Semicarbazonbindung gebunden sind, wie jene, welche zur Immobilisierung der Polypeptide verwendet wird.

4. Vorrichtung nach einem der Ansprüche 1 bis 3, **dadurch gekennzeichnet, dass** die Qualität der Funktionalisierung des Trägers (Dichtigkeit und Homogenität) durch seine Kapazität kontrolliert wird, eine durch eine α-Oxoaldehydfunktion derivatisierte, fluoreszierende peptidische Synthesesonde zu fixieren.

5. Vorrichtung nach einem der Ansprüche 1 bis 4, **dadurch gekennzeichnet, dass** sich die α-Oxoaldehyd- oder α-Oxoketonfunktion auf einem Spacerarm befindet.

6. Verfahren zur Herstellung einer Vorrichtung gemäß einem der Ansprüche 1 bis 5, **dadurch gekennzeichnet, dass** es die folgenden Schritte umfasst:
1- Einführen einer α-Oxoaldehyd- oder α-Oxoketonfunktion durch Synthese oder Modifikation einer natürlichen Funktion am N- oder C-terminalen Ende oder einer Seitenkette eines synthetischen oder natürlichen Polypeptids;
2- Funktionalisieren eines festen Trägers durch Semicarbazidgruppen;
3- Ablegen von Probepunkten von im Schritt 1 erhaltenen Polypeptiden auf dem in Schritt 2 funktionalisierten Träger unter pH- und Feuchtigkeitsbedingungen, welche die Umsetzung zwischen der α-Oxoaldehyd- oder α-Oxoketonfunktion und der Semicarbazidfunktion zur Bildung einer Semicarbazonbindung sicherstellen.

7. Verfahren gemäß Anspruch 6, **dadurch gekennzeichnet, dass** der Schritt 1 während der Synthese eines Polypeptids mit Hilfe eines Syntheseautomaten bewirkt wird.

8. Verfahren nach den Ansprüchen 6 oder 7, **dadurch gekennzeichnet, dass** der Schritt 1 des Weiteren das Einführen eines Spacerarms zwischen der letzten Aminosäure der Polypeptidsequenz und der α-Oxoaldehyd- oder α-Oxoketonfunktion umfasst.

9. Verfahren nach Anspruch 6, **dadurch gekennzeichnet, dass** der Schritt 1 durch Transaminierung einer N-terminalen Aminosäure eines natürlichen Proteins, das glykosiliert ist oder nicht, oder einem Fragment davon, durchgeführt wird.

10. Verfahren nach einem der Ansprüche 6 bis 9, **dadurch gekennzeichnet, dass** der Schritt 2
- eine Silanisierung des Trägers unter Einführung einer Aminfunktion,
- die Überführung der Aminfunktion in eine Isocyanatfunktion,
- die Umsetzung der Isocyanatfunktion mit einem Hydrazinderivat zur Bildung der Semicarbazidgruppe umfasst.

11. Verfahren gemäß einem der Ansprüche 6 bis 9, **dadurch gekennzeichnet, dass** der Schritt 2 durch eine einzige Umsetzung eines eine Semicarbazidgruppe tragenden Silans bewirkt wird.

12. Verfahren gemäß Anspruch 11, **dadurch gekennzeichnet, dass** die Semicarbazidgruppe vorzugsweise durch Fmoc geschützt ist.

13. Verfahren gemäß einem der Ansprüche 6 bis 12, **dadurch gekennzeichnet, dass** der Schritt 3
- die Herstellung von Polypeptidlösungen der Stufe 1 bis 10⁻³ oder 10⁻⁴M in einem 0,1M Acetatpuffer bei pH 5,5,
- ihre Verteilung in einem zu ihrer Entnahme geeigneten Behälter vom Typ Mikrotiterplatten,
- ihre Entnahme mit Hilfe eines "Spotters",
- und ihr Auftragen auf dem Semicarbazidträger;
- Inkubation über Nacht bei 37°C unter feuchter Atmosphäre
- und Waschen und Sättigen der nichtspezifischen Reaktionsstellen.

14. Verwendung einer Vorrichtung gemäß den Ansprüchen 1 bis 5 wie Polypeptidchips als Diagnosetool.

15. Verwendung der Vorrichtung nach Anspruch 14, **dadurch gekennzeichnet, dass** sie den Nachweis von Antworten vom Typ Antigen-Antikörper durch die in-vitro-Verwendung von fluoreszierenden, radioaktiven oder chemischen Markern umfasst, wie bei nichtminiaturisierten Diagnosetest.

16. Verwendung der Vorrichtungen gemäß einem der Ansprüche 1 bis 5 wie Polypeptidchips für das Trennen von Molekülen.

17. Verwendung der Vorrichtungen gemäß einem der Ansprüche 1 bis 5 wie Polypeptidchips zur Analyse des Verhältnisses zwischen Molekülen vom Typ Ligand-Rezeptor.

18. Verwendung einer Vorrichtung gemäß einem der Ansprüche 1 bis 5 wie Polypeptidchips für den miniaturisierten Nachweis von strukturell oder funktionell komplementären Molekülen.
